# EUROPEAN PATENT SPECIFICATION

(11) **EP 1 883 414 B1**
(45) Date of publication and mention of the grant of the patent: **13.06.2012**
(21) Application number: 06740374.1
(22) Date of filing: 31.03.2006
(51) Int. Cl.: A61K 35/12, A61K 35/14

(54) **PREVENTING REJECTION OF TRANSPLANTED TISSUE USING REGULATORY T CELLS**
VERHINDERUNG DES ABSTOSSES VON TRANSPLANTIERTEM GEWEBE UNTER VERWENDUNG REGULATORISCHER T-ZELLEN
PROCEDE DE PREVENTION DU REJET D'UN TISSU TRANSPLANTE EN UTILISANT DE CELLULES REGULATOIRES

(30) Priority: 01.04.2005 US 667494 P
(43) Date of publication of application: 06.02.2008
(60) Divisional application: 10010713.5
(73) Proprietor: UNIVERSITY OF SOUTHERN CALIFORNIA, Los Angeles, CA 90033 (US)
(72) Inventor: HORWITZ, David, Santa Monica, California 90402 (US)
(74) Representative: Kiddle, Simon John
(86) International application number: PCT/US2006/012261
(87) International publication number: WO 2006/107850

(56) References cited:
- WO-A2-01/77299
- WO-A2-03/066072
- COBBOLD S P; GRACA L; LIN CHUN-YEN; ADAMS E; WALDMANN H: "Regulatory T cells in the induction and maintenance of peripheral transplantation tolerance." TRANSPLANT INTERNATIONAL : OFFICIAL JOURNAL OF THE EUROPEAN SOCIETY FOR ORGAN TRANSPLANTATION FEB 2003, vol. 16, no. 2, February 2003 (2003-02), pages 66-75, XP002421528 ISSN: 0934-0874
- TAAMS LEONIE ET AL: "Immune regulation by CD4+CD25+ regulatory T cells: implications for transplantation tolerance" TRANSPLANT IMMUNOLOGY, ELSEVIER, vol. 11, no. 3-4, July 2003 (2003-07), pages 277-285, XP002391307 ISSN: 0966-3274
- HORWITZ D A ET AL: "THE POTENTIAL OF HUMAN REGULATORY T CELLS GENERATED EX VIVO AS A TREATMENT FOR LUPUS AND OTHER CHRONIC INFLAMMATORY DISEASES" ARTHRITIS RESEARCH, CURRENT SCIENCE, LONDON,, GB, vol. 4, no. 4, 2002, pages 241-246, XP009032180 ISSN: 1465-9905
- BRYAN JOHNSON: "Alloantigen-specific regulatory T cells" BLOOD, vol. 103, no. 11, 1 June 2004 (2004-06-01), page 4000, XP002421529
- JIANG SHUIPING; CAMARA N; LOMBARDI G; LECHLER R I: "Induction of allopeptide-specific human CD4+CD25+ regulatory T cells ex vivo" BLOOD, vol. 102, no. 6, 15 September 2003 (2003-09-15), pages 2180-2186, XP002421530 ISSN: 0006-4971

## Description

### TECHNICAL FIELD

The present invention relates to medical uses for preventing rejection of transplanted tissue in which recipient alloactivated regulatory T cells generated *ex vivo* are introduced into the recipient before transplantation. Donor antigen is introduced into the recipient after transplantation to boost recipient regulatory T cells.

### BACKGROUND OF THE INVENTION

Experimental autoimmune and transplant models have shown that several mechanistic approaches that include clonal deletion, anergy, and effector cell regulation can alter T cell alloreactivity and drive the immune system towards one of unresponsiveness (Elster, E. A., et al., Transpl Immunol 13:87 (2004)). There is increasing evidence that CD4+ cells that constitutively express CD25, the alpha chain of the IL-2 receptor, not only have an important role in preventing autoimmunity, but can also prevent graft rejection (Sakaguchi, S., et al., Immunol Rev 182:18 (2001); Piccirillo, C. A. and Shevach, E. M., Semin Immunol 16:81 (2004); Cohen, J. L., et al., J Exp Med 196:401 (2004)). CD4+CD25+ cells with a typical phenotype and suppressive effects occur naturally (Sakaguchi, S., et al., Immunol Rev 182:18 (2001); Piccirillo, C. A. and Shevach, E. M., Semin Immunol 164:81 (2004)), or can be induced peripherally (Yamagiwa, S., et al., J Immunol 166:7282 (2001); Chen, Z.M., et al., Blood 101:5076 (2003)). Endogenous CD4+CD25+ cells can be expanded (Godfrey, W. R., et al., Blood 104:453 (2004)) so that they can be used in clinical trials. Prior studies have shown that peripheral CD4+CD25+ cells that prevent graft rejection can be induced indirectly using non-depleting CD4 and CD8 monoclonal antibodies, costimulatory inhibitors, or immunosuppressive drugs (van Maurik, A., et al., J Immunol 169:5401 (2002); Graca, L., Thompson, et al., J Immunol 168:5558 (2002); Taylor, P. A., et al., J Exp Med 193:1311 (2001); Gregori, S., et al., J Immunol 167:1945 (2001)).

The combination of interleukin 2 (IL-2) and transforming growth factor beta (TGF-β) can induce both CD4+ and CD8+ cells to develop potent immunosuppressive activity (Yamagiwa, S., et al., J Immunol 166:7282 (2001); Gray, J. D., et al., J Exp Med 180:1937 (1994); Zheng, S. G., et al., J Immunol 169:4183 (2002); Horwitz, D. A., Semin Immunol 16:135 (2004); Zheng, S. G., et al., J Immunol 172:1531 (2004); Zheng, S. G., et al., J Immunol 172:5213 (2004)). These cytokines induced naive human, alloantigen-stimulated, peripheral blood CD4+ cells to become CD25+ regulatory cells with a surface phenotype and cytolcine-independent suppressive effects indistinguishable from natural CD4+CD25+ cells (Yamagiwa, S., et al., J Immunol 166:7282 (2001)). Moreover, these CD4+CD25+ Treg cells are able to induce other CD4+ cells to develop cytokine-dependent suppressive activity *in vitro* (Zheng, S. G., et al., J Immunol 172:5213 (2004)).

H-2^{d} anti-H-2^{b} Treg cells generated in the presence of IL-2 and TGF-β *ex vivo* have been used without other immunosuppression to prevent rejection of H-²b heart transplants. In previous experiments, CD4+ and CD8+ Treg cells were generated by stimulating DBA/2 (H-2^{d}) mouse T cells with C57BL/6 (H-2^{b}) alloantigens in the presence of IL-2 and TGF-β. These Tregs were antigen-specific and prevented a chronic graft-versus-host disease with features of systemic lupus erythematosus in (DBA/2 x C57BL/6) F1 mice. Moreover, a single injection of these cells in mice with established disease doubled their survival (Zheng, S. G., et al., J Immunol 172:1531 (2004)).

### SUMMARY OF THE INVENTION

Peripheral blood mononuclear cells (PBMC) from a recipient are stimulated with one or more donor antigens such as donor PBMCs or other donor cells such as spleen cells in the presence of certain messenger proteins. This results in the formation of recipient regulatory T cells that are alloactivated by the donor antigen. These cells are also referred to as donor alloactivated recipient regulatory T cells or recipient Treg cells. Prior to treatment, the PBMCs may be further purified to produce populations of CD4⁺ T cells, CD8⁺ T cells and/or NK-T cells.

Accordingly, in a first aspect, the present invention provides an *ex vivo* population of donor alloactivated regulatory recipient T cells (Tregs) for use in a method for preventing rejection of donor tissue in a recipient, wherein the method comprises:
(a) producing, *ex vivo,* a population of donor alloactivated regulatory recipient T cells (Tregs);
(b) introducing a first dose of said recipient Tregs into said recipient;
(c) transplanting donor tissue into said recipient; and
(d) introducing donor antigen into said recipient after transplantation of the donor tissue.

In a further aspect, the present invention provides the use of an *ex vivo* population of donor alloactivated regulatory recipient T cells (Tregs) for the preparation of a medicament for preventing rejection of donor tissue in a recipient, wherein the administration pattern of the medicament comprises (a) introducing a first dose of said recipient Tregs into said recipient and (b) introducing donor antigen into said recipient after transplantation of the donor tissue.

In a further aspect, the present invention provides an *ex vivo* population of donor alloactivated regulatory recipient T cells (Tregs) for preventing rejection of donor tissue in a recipient, wherein the administration pattern of the medicament comprises (a) introducing a first dose of said recipient Tregs into said recipient and (b) introducing donor antigen into said recipient after transplantation of the donor tissue. . The preferred recipient is a human.

### BRIEF DESCRIPTION OF THE DRAWINGS

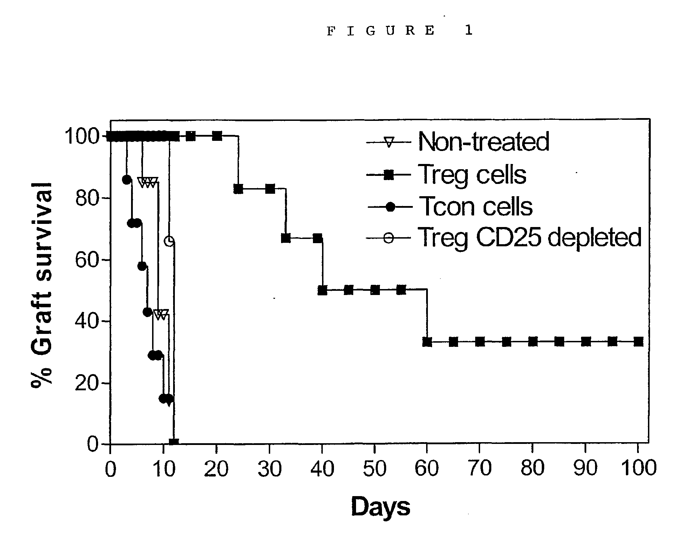

Figure 1 demonstrates that donor anti-H-2^{b} -specific Tregs induced *ex-vivo* with TGF-β result in long term survival of mismatched allogeneic heart transplants. Regulatory T cells (Tregs) were generated by stimulating DBA/2 (D2, H-2^{d}) T cells with irradiated C57BL/6 (B6, H-2^{b}) non-T cells and IL-2 in the presence of TGF-β (2 ng/ml) for 5-6 days. T cells stimulated with IL-2 only served controls (Tcon). D2 mice that received B6 heart transplants were injected with 10x10⁶ Treg, Tcon, or Treg depleted CD25+ cells IV on days -1 and +5. Six mice were in each group.

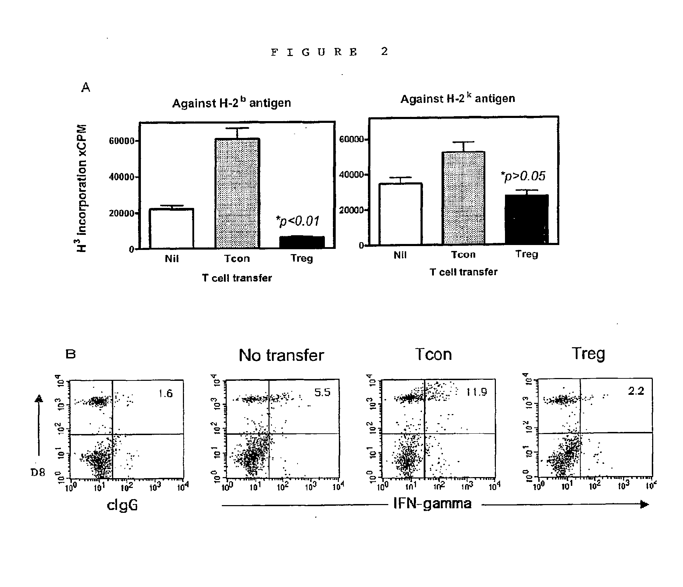

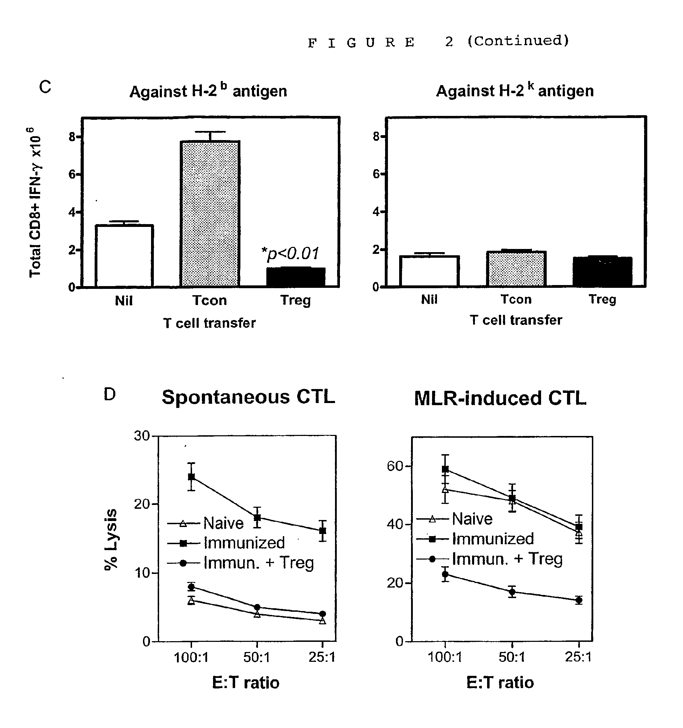

Figure 2 demonstrates that transferred anti-H-2^{b} Tregs induce alloantigen-specific T cell non-responsiveness. Groups of 4 naive DBA/2 mice were injected IV with or without 10x10⁶ D2 Tcon, Treg cells generated as described in Figure 1. Another non-injected group served as additional controls. One month later the animals were sacrificed and splenic T cells were alloactivated with B6 or third party, C3H (H-2^{k}) stimulator cells *in vitro* for 4 days. **A.** Proliferative activity (mean counts per minute ± SEM). P values indicate significant differences between mice that received Treg cells and mice that received Tcon cells or no transfer (Nil). **B.** Example of percentages of IFN-γ-producing CD8+ cells in response to H-2^{b} antigen determined by flow cytometry. **C.** Number of IFN-γ-producing splenic CD8+ cells against H-2^{b} and H-2^{k} antigens. P values were determined as described above. The experiment was repeated with similar results. **D.** DBA/2 mice were immunized with 10x10⁶ B6 splenocytes injected intravenously with or without 10x10⁶ D2 Treg cells. Unimmunized mice served as controls. One month later, fresh splenic T cells were tested for anti-H-2^{b} CTL activity, or alloactivated with B6 stimulator cells. The cells from the MLR cultures were re-counted and assayed for CTL activity at the indicated effector to target ratio. Values indicate the mean ± SEM of 6 mice. The experiment was repeated with similar results.

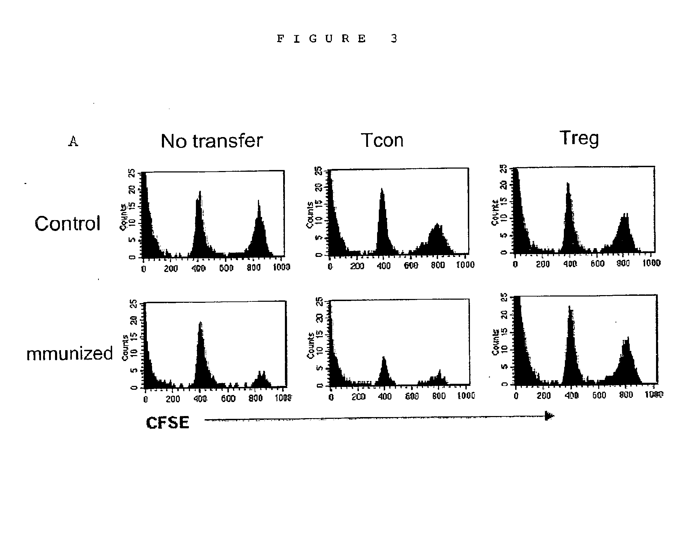

Figure 3 demonstrates that transferred anti-H-2^{b} Tregs results in the reduced cytotoxicity activity *in vivo.* The experimental design is similar to that described in Figure 1. Naive DBA/2 mice were injected IV with or without 10x10⁶ D2 Tcon, Treg cells or no cells (N=8/group). On the third week, 4 mice (one half) of each group were injected with 10x10⁶ B6 splenocytes. To assess the immune response to B6 cells, one week later all mice received 10x10⁶ B6 splenocytes brightly labeled with CFSE and a similar number of dimly CFSE labeled C3H splenocytes. The animals were sacrificed 2 hours later and splenic cells examined for intensity of CFSE staining by flow cytometry. Results are expressed as the percentage of killing B6 or third party C3H CFSE stained cells in immunized animals compared with that in unimmunized animals.

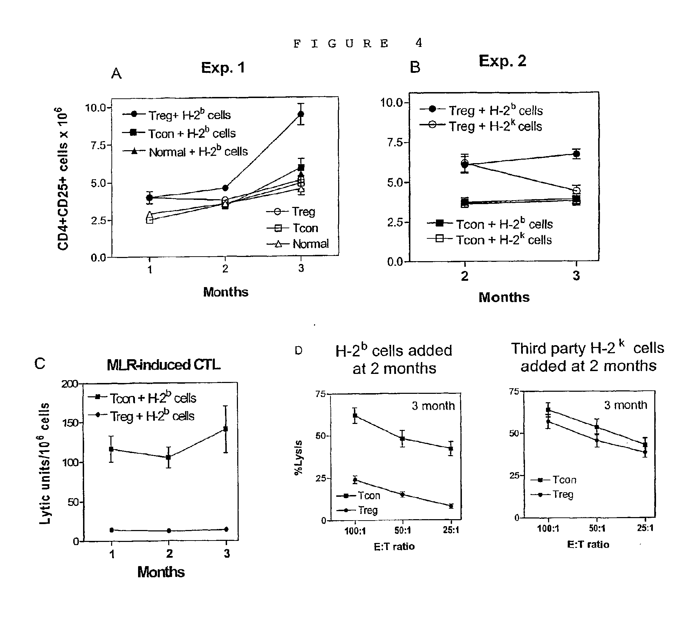

Figure 4 demonstrates that continuous antigen-stimulation results in a progressive increase in CD4+CD25+ cells and maintenance of tolerogenic effects. **A.** Groups of 6 mice received a single injection of 10 x10⁶ D2 Treg (circles), Tcon (squares) or no cells (triangles). Those with filled symbols also were also injected with 10 x10⁶ H-2^{b} B6 irradiated splenocytes every two weeks. Those with empty symbols were not boosted with alloantigen. Splenic CD4+CD25+ cell numbers were determined each month by cell counts and flow cytometry in mice. Note the antigen-dependent increase in CD4+CD25+ cells in mice that received alloantigen. **B.** Two months after the single dose of Tcon or Tregs, some groups continued to receive specific antigen, but others were injected with third party H-2^{k} (C3H) antigen followed by another injection two weeks later. Note that the increased numbers CD4+CD25+ cells in mice given Tregs and boosted with H-2^{b} cells decreased to baseline levels when H-2^{K} cells were given instead. **C.** D2 mice received a single injection IV of 10x10⁶ syngeneic Tcon or Tregs, and 10x10⁶ irradiated B6 splenocytes every two weeks to provide a continuous source of antigen. One, 2, and 3 months post-injection, splenic T cells were tested for CTL activity in an allo-MLR with results in lytic units expressed as the mean ± SEM. One lytic unit is the number of lymphocytes required to give 30% lysis. Six mice per group were examined at each time point. **D.** The tolerogenic response was antigen-dependent. Using the protocol described in the description of Figure 4B, H-2^{k} cells were substituted for H-2^{b} cells at 2 months and the animals were tested for anti-B6 CTL activity one month later. Note the loss of CTL activity at this time that is associated with the cessation of vH-2^{b} antigen stimulation.

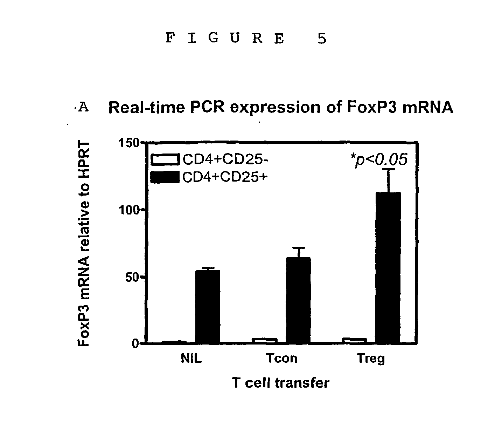

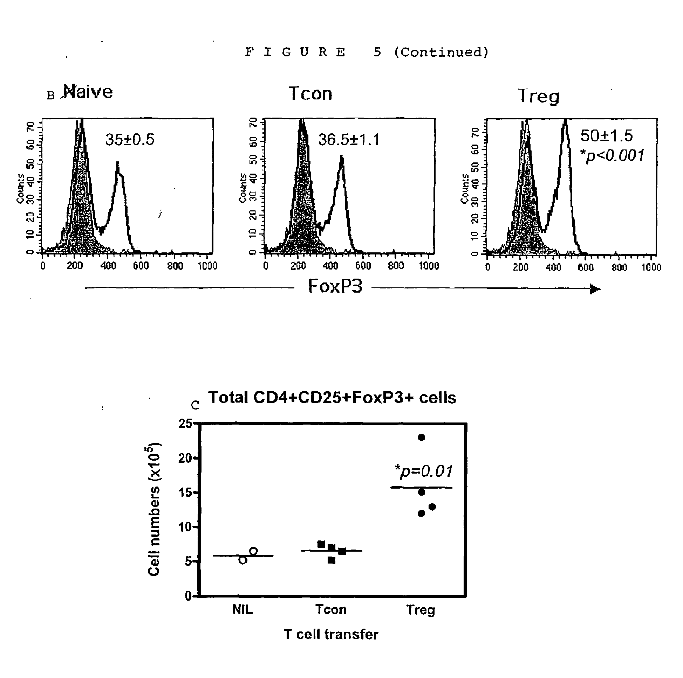

Figure 5 demonstrates that CD4+CD25+ cells express increased levels of FoxP3 mRNA and protein. The experimental design is similar to that shown in Figure 4. Groups of 4 D2 mice received a single injection of 10 x10⁶ syngeneic Treg, Tcon. Another 2 mice received no T cells. All mice shown were injected with 10 x10⁶ H-2^{b} B6 irradiated splenocytes every two weeks. Splenic CD4+CD25+ cell numbers of each mouse was determined at two months by cell counts and FACS staining. **A.** Splenic CD4+CD25+ cells were positively selected from individual mice by immunomagnetic beads, and FoxP3 mRNA was quantified by real-time PCR. The numbers shown are the mean ± SEM of each group. **B.** A representative example of FoxP3 protein expression in these CD4+CD25+ cells was determined by staining with anti-mouse FoxP3 antibody. **C.** The numbers shown indicate the mean ± SEM of total CD4+CD25+FoxP3+ cells of each group.

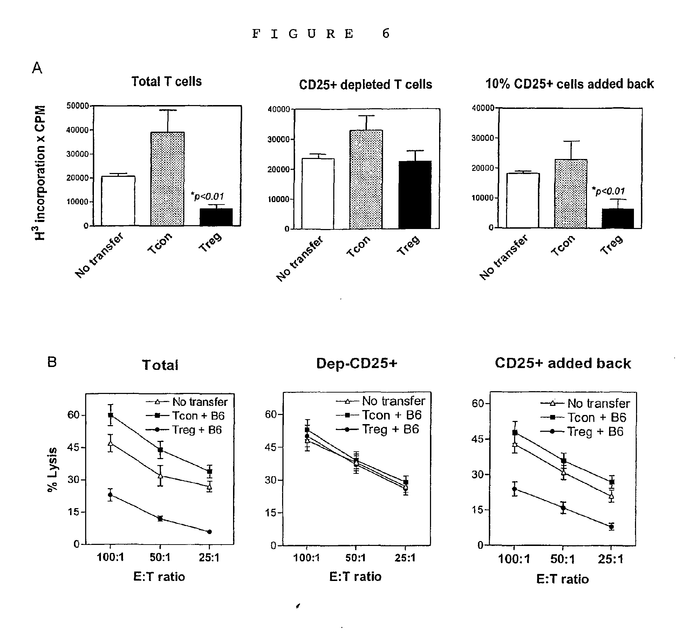

Figure 6 demonstrates that CD4+CD25+ cells are responsible for tolerance to donor alloantigens. **A.** Splenic T cells, T cells depleted of CD25 prior to the culture, and CD25 depleted T cells with 10% of these CD25+ cells added back, were prepared from mice that had received a single injection of Tcon, Tregs, or no injection (No transfer) three months previously. These D2 T cells were alloactivated with B6 stimulator cells and tested for proliferative ability. Note that CD4+CD25+ cells were responsible for the suppressive effects. **B.** Each T cell preparation was also tested for anti-B6 CTL activity and these suppressive effects were also dependent on CD25+ cells. Values shown are representative of the 6 mice in each group.

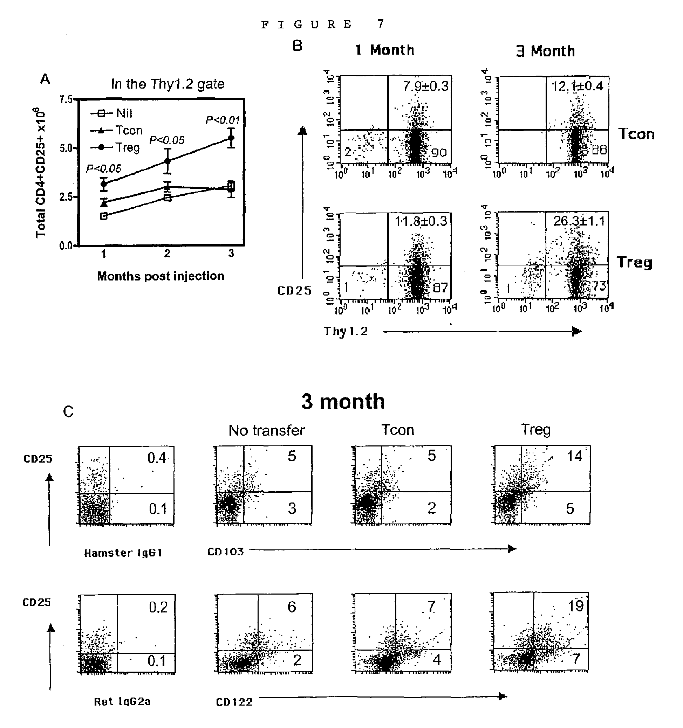

Figure 7 demonstrates that the transferred Tregs increase recipient CD4+CD25+ cells that express CD 103, CD 122 and GITR. To distinguish transferred T cells from recipient T cells, anti-H-2^{d} Tregs and Tcon were prepared from cells from B6 Thy1.1 mice and 8x10⁶ cells transferred to congenic Thy 1.2 mice. Using the repeated stimulation protocol described above, the numbers of CD4+CD25+ cells and phenotype was assessed sequentially for 3 months. **A.** Total numbers of recipient Thy 1.2 CD4+CD25+ cells each month. **B.** Flow cytometry profile at 1 and 3 months of splenic cells stained with CD4, Thy1.2 and CD25. The cells shown were gated on CD4+ cells. **C.** Percentage of CD4 cells expressing CD25, CD 122 and CD 103 in the Thy 1.2 gate.

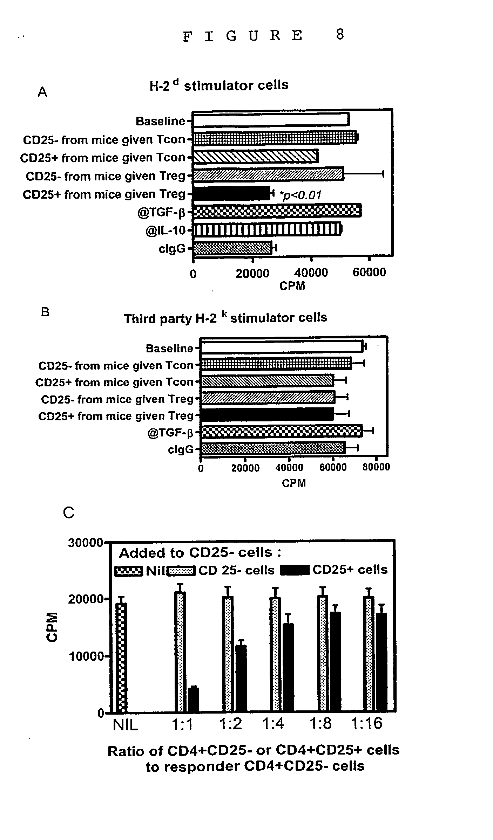

Figure 8 demonstrates that transferred Tregs induce recipient CD4+ cells to become antigen-specific suppressor cells. **A.** Three months after transfer of Tcon or Tregs, splenic CD4+CD25+ and CD4+CD25- cells were obtained by cell sorting and their suppressive effects on the allogeneic response of fresh, syngeneic CD4+CD25-cells to H-2^{d}, indicated as baseline. The ratio of sorted CD4+ cells to responder CD4+CD25- cells was 1:6 to dilute out the non-specific suppressive activity of CD4+CD25+ cells (**see below**). The effect of neutralizing anti-IL-10 (10µg/ml) or TGF-β (10µg/ml) antibodies on the suppressive activity of CD4+CD25+ cells is also shown. **B.** Lack of suppression following stimulation by third party (H-2^{k}) cells. Results are expressed as mean cpm ± SEM of triplicate wells (n = 6 mice/group). **C.** Suppressive activity of naive CD4+CD25+ cells. CD4+ CD25+ and CD25- cells from naive mice were prepared by cell sorting and assayed for their suppressive effects on the response of CD4+CD25- cells to H-2^{b} stimulator cells.

### DETAILED DESCRIPTION OF THE INVENTION

Donor alloactivated recipient regulatory T cells ("recipient Treg cells" or "Treg cells") are used with donor antigen to prevent rejection of transplanted tissue. As described below, recipient Tregs are prepared *ex vivo* by culturing recipient PBMCs with donor antigen. The recipient Tregs, with or without donor antigen, are introduced into the recipient prior to transplantation of donor tissue. Thereafter, donor antigen, alone or in combination with recipient Tregs, is administered to the recipient. This treatment prevents rejection of the transplanted tissue. It is to be understood that preventing rejection includes complete prevention as well as delayed rejection compared to transplantation without the use of donor antigen after transplantation.

Methods for making recipient Treg cells are well known in the art. (See, e.g., PCT Publication WO/01/77299 published October 18, 2001. Briefly, the recipient PBMCs are cultured with donor antigen in the presence of a regulatory composition. The culturing can last up to about 5-7 days after which the recipient Treg cells start to loose immunosuppressive function.

An alternate approach uses two stage culturing of the recipient PBMCs as disclosed in WO 2006/127152 (US 60/668,676).
Briefly, the methods involve: (1) removing cells from a patent and treating them for 24-48 hours with a first regulatory composition comprising TGF-β and optionally a mitogen and/or cytokine, (2) removing the first regulatory composition followed by (3) culturing the cells with a second regulatory composition comprising a cytokine. T regs produced by treatment with these two regulatory compositions produce a higher ratio of suppressor cells to helper cells as compared to treatment with TGF-β and cytokine for 5-6 days.

By "regulatory composition" herein is meant a composition that can cause the formation of regulatory T cells when cultured with recipient PBMCs and donor antigen. Generally, these compositions comprise TGFβ alone or in combination with a cytokine such as IL-2, IL-4, IL-10, IL-15 and/or TNIα. IL-2 is the preferred cytokine.

Suitable regulatory compositions may also include T cell activators such as anti-CD2, including anti-CD2 antibodies and the CD2 ligand, LFA-3, and mixtures or combinations of T cell activators such as Concanavalin A (Con A) or staphylococcus enterotoxin B (SEB). A preferred regulatory composition for antibody suppression is a mixture containing a T cell activator, IL-2 and TGF-β. In a preferred embodiment, anti-CD3 or anti-CD28 are used in combination with TGFβ and cytokine.

By "transforming growth factor -β" or "TGF-β" herein is meant any one of the family of the TGF-βs, including the three isoforms TGF-β1, TGF-β2, and TGF-β3; see Massague, J. (1980), J. Ann. Rev. Cell Biol 6:597. Lymphocytes and monocytes produce the β1 isoform of this cytokine (Kehrl, J.H. et al. (1991), Int J Cell Cloning 9: 438-450). The TFG-β can be any form of TFG-β that is active on the mammalian cells being treated. In humans, recombinant TFG-β is currently preferred. A preferred human TGF-β can be purchased from Genzyme Pharmaceuticals, Farmington, MA. In general, the concentration of TGF-β used ranges from about 2 picograms/ml of cell suspension to about 5 nanograms, with from about 10 pg to about 4 ng being preferred, and from about 100 pg to about 2 ng being especially preferred, and 1 ng/ml being ideal.

IL-2 can be any form of IL-2 that is active on the mammalian cells being treated. In humans, recombinant IL-2 is currently preferred. Recombinant human IL-2 can be purchased from R & D Systems, Minneapolis, MN. In general, the concentration of IL-2 used ranges from about 1 Unit/ml of cell suspension to about 100 U/ml, with from about 5 U/ml to about 25 U/ml being preferred, and with 10 U/ml being especially preferred. In a preferred embodiment, IL-2 is not used alone.

In some embodiments it is desirable to use a mitogen to activate the cells; that is, many resting phase cells do not contain large amounts of cytokine receptors. The use of a mitogen such as Concanavalin A or staphylococcus enterotoxin B (SEB) can allow the stimulation of the cells to produce cytokine receptors, which in turn makes the present invention more effective. When a mitogen is used, it is generally used as is known in the art, at concentrations ranging from 1 µg/ml to about 10 µg/ml is used. In addition, it may be desirable to wash the cells with components to remove the mitogen, such as α-methyl mannoside, as is known in the art.

In a preferred embodiment, T cells are strongly stimulated with mitogens, such as anti-CD2, anti-CD3, anti-CD28 or combinations of these monoclonal antibodies especially anti-CD3 and anti-CD28. Repeated stimulation of the T cells with or without TGF-β in secondary cultures may be necessary.

A subset of CD4+ T cells that express CD25, the alpha chain of the IL-2 receptor, can induce and maintain T cell non-responsiveness to donor alloantigens and, therefore, have attractive therapeutic potential in solid organ transplantation. Peripheral CD4+ cells alloactivated with IL-2 and TGF-β *ex vivo* express the transcription factor FoxP3, and become potent antigen-specific suppressor cells. The transfer of TGF-β induced regulatory T cells co-incident with transplantation of a histo-incompatible heart resulted in extended allograft survival. To account for this result, non-transplanted mice were injected with a single dose of regulatory T cells and transferred donor cells every two weeks to mimic the continuous stimulation of a transplant. Increased splenic CD4+CD25+ cells were observed that were of recipient origin. These cells rendered the animals non-responsive to donor alloantigens by an antigen-specific and cytokine-dependent mechanism of action. Both the increased number of CD4+CD25+ cells and their tolerogenic effect were dependent upon continued donor antigen boosting. Thus, regulatory T cells generated *ex vivo* can act like a vaccine that generates host suppressor cells with the potential to protect MHC mismatched organ grafts from rejection.

As used herein, a "donor antigen" can be any antigen derived from a donor that (1) induces the formation of a recipient's regulatory T cells or (2) boosts the recipient Treg population when administered to the recipient. Examples of donor antigens include donor cells such as spleen cells, peripheral blood mononuclear cells, bone marrow cells, lymph node cells, tonsil cells and tissue extracts containing histocompatibility antigens. Other examples of donor antigens include peptides and proteins derived from the donor's major histocompatibility complex (MHC) that are produced recombinantly as well as peptides and proteins derived from related MHCs

After the MHC antigens of the donor's cells are typed, donor PBMC or histocompatible PBMC, or preferably, recombinant MHC peptides shared by the donor are cultured with recipient purified CD4+ and/or CD8+ cells in sufficient quantities to activate the recipient T cells. Activation is defined as the expression of specific surface markers or the proliferation of these cells as assessed by standard methods known to those familiar with the art. The donor cells can be used directly, or converted to antigen-presenting dendritic cells by standard methods. Ratios of donor cells to recipient cells vary between 0.01:1 (for dendritic cells) to 1:1 (for irradiated donor non-T cells). The number of Tregs transferred can range from 10⁵ to 10⁸ cells per kg. The number of donor cells used to sustain Treg activity can range from 10⁴ to 10⁷ cells per kg. Donor B cells or histocompatible B cells from a related donor can be greatly expanded by EBV transformation and used as the source of donor antigen.

As used herein, "donor tissue" is any tissue that can be transplanted from one individual to another, preferably within the same species. Donor tissue includes kidney, heart, lung, liver, intestine, pancreas and pancreatic islet cells. The preferred recipient is human.

### EXAMPLES

Tregs induced *ex vivo* can substantially delay rejection of heart allografts in non-lymphopenic mice using allogeneic spleen cell immunization. The transfer of TGF-β induced Tregs have antigen-specific tolerogenic effects in these mice. These cells induced recipient CD4+ cells to become CD4+CD25+ cells that are responsible for the T cell non-responsiveness. In order to sustain these CD4+CD25+ cells and their tolerogenic effects, continuous boosting of allogeneic donor cells was required.

### Materials and Methods

### Animals

Male C57BL/6 (B6, H-2^{b}), DBA/2 (D2, H-2^{d}), and C3H (H-2^{k}) mice were purchased from the Jackson Laboratory (Bar Harbor, ME). Animals eight to ten weeks of age were used as graft donors, recipients, and controls. All mice were housed in conventional facilities at University of Southern California using animal care protocols approved by the IACUC of University of Southern California.

### Antibodies and reagents

The following Abs were obtained from eBioscience (San Diego, CA): Anti-CD3-PE (145-2011), anti-CD4-FITC (RM4-5), anti-CD4-PE (GK1.5), anti-CD8-PE (53-6.7), anti-CD25-PE (PC61), anti-CTLA-4-PE (UC10-4B9), anti-CD122-PE (51-14), anti-CD103-FITC (2E7), anti-IFN-γ (XMG1.2), anti-FoxP3 (FJK-16S), anti-Thy1.1-PE (A20) and anti-Thy1.2-FITC (104). The anti-H-2^{d}-FITC (SF1-1.1) and anti-H-2^{b} (AF6-88.5) came from BD Pharmingen (San Diego, CA). Isotype controls Abs were also obtained from eBioscience and BD Pharmingen. Anti-GITR-biotin (BAF524), anti-IL-10 (mAb417); anti-TGF-β (mAb240) and matched isotype control abs were obtained fromR&D Systems (Minneapolis, MN).

### Cell preparations and adoptive transfer

T cells were prepared from D2 spleen cells by collecting nylon wool column non-adherent cells (Zheng, S. G., et al., J Immunol 172:153 (2004)). The T enriched cells (1.5 x 10⁶ per ml) were stimulated with similar numbers of irradiated (2000 rad) B6 nylon adherent, non-T cells for 5-6 days in 24 well plates (2ml/well) (Becton Dickinson Labware, Franklin Lakes, New Jersey) in AIM V (InVitrogen, Carlsbad, California) serum-free medium with additives (Zheng, S. G., et al., J Immunol 172:1531 (2004)). Some wells contained TGF-β1 (2ng/ml) and rhuIL-2 (15 to 20 units/ml) (R&D Systems) or IL-2 only. Groups of 6 D2 mice were injected intravenously 1 day before and 5 days after receiving B6 heart allograft with ten million viable alloactivated T cells primed with IL-2 and TGF-β (Treg) and others with IL-2 only (Tcon), or with Treg depleted of CD25+ cells with immunomagnetic beads (Miltenyi). These preparations contained approximately 10% residual B6 stimulator cells.

### Heterotopic heart transplantation

Abdominal vascularized heterotopic heart transplants were performed essentially as previously described (Cramer, D. V., et al. In Handbook of Animal Models in Transplantation Research, 1st edn., p. 149-160. CRC Press, Boca Raton, LA (1993)). Rejection was defined as complete cessation of a palpable cardiac contraction and confirmed by visualization after laparotomy. Recipients with grafts surviving >100 days were considered as permanent and were sacrificed for *in vitro* experiments.

### Assays of T cell function

The proliferative activity of T cells to alloantigens was measured using a standard one way mixed lymphocyte culture with 2 x 10⁵ T cells and an equal number of irradiated allogeneic non-T cells in a 96 well flat bottomed plate using RPMI 1640 culture medium and 10% fetal calf serum with additives as described previously (Zheng, S. G., et al., J Immunol 172:1531 (2004)). Proliferation was measured after 4-5 days as uptake of ³H-thymidine in triplicate cultures. In order to analyze the IFN-γ-producing cells, intracellular cytokine staining was performed as described previously (Zheng, S. G., et al., J Immunol 172:5213 (2004)). In cultures used to assess the suppressive activity of CD4+CD25+ cells, the ratio of primed cells to CD4+CD25- responder cells was 1:6. T cell cytotoxic activity was assessed using various ratios of effector cells to target cells (Chromium-labeled Con A blasts) in a standard 4 hour assay as described previously. Values indicate the mean ± SEM of triplicate cultures and in some experiments expressed as the lytic units per 10⁶ cells (Yamagiwa, S., et al., J Immunol 166:7282 (2001)). Lytic units were based on the number of effector cells required to kill 30% of the target cells.

### FoxP3 expression by real-time RT-PCR

Total RNA was prepared with TRIzol LS reagent (Invitrogen). First strand cDNA was synthesized using Omniscript TR kit (Qiagen, Valencia, CA) with random hexamer primers (Invitrogen). Real-time PCR was performed with a LightCycler (Roche, Mannheim, Germany), and message levels were quantified using the LightCycler Fast Start DNA Master SYBR Green I Kit (Roche), according to the manufacturer's instructions. Amplification was conducted for 45 cycles. The recovered PCR product and amplicon were checked by agarose gel electrophoresis for a single band of the expected size. The samples were run in triplicate and the relative expression of FoxP3 was determined by normalizing expression of each target to hypoxanthine guanine phosphoribosyl transferase (HPRT). Primer sequences were as follows: HPRT 5'-TGA AGA GCT ACT GTA ATG ATC AGT CAA C-3' and 5'-AGC AAG CTT GCA ACC TTA ACC A-3'; FoxP3 primers: 5'-CCC AGG AAA GAC AGC AAC CTT-3' and 5'-TTC TCA CAA CCA GGC CAC TTG-3' (Hori, S., Nomura, T., and Sakaguchi, S. Science 299:1057 (2003)).

### In vivo cytototoxic T cell activity

Groups of 8 DBA/2 mice were injected intravenously with 10⁷ Treg or T con cells generated *ex vivo* as described above. Another group was not injected. Three weeks later, four mice from each group were injected with 10⁷ C57BL/6 splenocytes (immunized) or served as controls. *In vivo* cytotoxic T cell activity was assessed at week four using an assay modified from that described by Suvas and co-workers (Suvas, S., et al., J Exp Med 198:889 (2003)). Splenic target cells from C57BL/6 or C3H mice were labeled with high (2.5 mM) or low (0.25 mM) concentrations of CFSE. Equal numbers (10⁷) of donor-specific and third party target cells were mixed together and adoptively transferred intravenously into control and immunized DBA/2 mice. Splenocytes were collected at 1, 2 or 4h after adoptive transfer from recipient mice, erythyrocytes were lysed, and cell suspensions were analyzed by flow cytometry. Each population could be distinguished by their respective fluorescence intensity. Assuming that the number of C57BL/6 target cells that migrated to the spleen in unimmunized mice is equivalent to the number of splenic C57BL/6 target cells injected in immunized mice, the percentage of killing of target cells in the immunized animals was determined as: % Killing = [(Percentage of CFSE⁺ subset in the control mice - percentage of CFSE⁺ in the immunized mice) ÷ Percentage CFSE⁺ in the control mice] x 100.

### Statistical analysis

Analysis for statistically significant differences between groups of mice was performed by t test and Wilcoxon test survival curves with the log rank test using GraphPad PRISM software (GraphPad, San Diego, CA).

### Results

### Treatment with regulatory T cells generated ex vivo markedly prolongs the survival of heart allografts

Since we have shown that TGF-β induces both CD4+ and CD8+ cells to become suppressor cells (Yamagiwa, S., et al., J Immunol 166:7282 (2001); Gray, J. D., et al., J Exp Med 180:1937 (1994), and others have described CD8+ regulatory cells that express FoxP3 with functional properties similar to CD4+CD25+ regulatory T cells (Xystrakis, E., et al., Blood 104:3294 (2004)), we generated Tregs from unseparated T cells. Our objective was to learn whether a combination of CD4+ and CD8+ Tregs induced *ex vivo* with TGF-β used as sole therapy could prolong survival of totally MHC mismatched heart allografts. After culture of DBA/2 (H-2^{d}) T cells with irradiated C57BL/6 (H-2^{b}) spleen cells for 5 to 6 days with IL-2 and TGF-β, we recovered approximately the starting number of T cells in cultures with TGF-β, and 50% of T cells in cultures without TGF-β. In cultures with IL-2 and TGF-β, 60 ± 4.1 % of CD4+ cells expressed CD25 and 55 ± 4.8% of CD8+ cells expressed this marker. These cells are called Treg. In cultures without TGF-β these values were 45 ± 3.4% and 49 ± 4.1 % respectively. These cells are called Tcon. Of the 10 million cells injected into recipient mice, Treg preparations contained 3.4 ± 0.3 x 10⁶ CD4+CD25+ cells and 2.1 ± 0.2 x 10⁶ CD8+CD25+ cells. Tcon preparations contained 2.1 ± 0.2 x 10⁶ CD4+CD25+ cells and 1.6 ± 0.15 x 10⁶ CDS+CD25+ cells, respectively.

All hearts from B6 mice that were transplanted into D/2 recipients were rejected within 11 days of transplantation. Transfer of 10 million Treg at days -1 and +5 resulted in extended survival of B6 heterotopic heart transplants up to 100 days, at which point the experiment was terminated. By contrast, rejection was accelerated in D2 mice that received similar numbers of Tcon (Fig.1). The extended survival was dependent on CD25+ cells, since depletion of this subset completely abolished all suppressive effects.

### The transfer of Treg cells results in the antigen-specific tolerance in the recipients

We next developed a model designed to investigate the mechanism of action of the long term suppressive effects. D2 mice were given a single injection of 10⁷ Treg or Tcon cells. One month later they were tested for T cell responsiveness to donor alloantigen. Figure 2 shows that animals injected with Tcon proliferated vigorously to H-2^{b} antigen. By contrast, animals injected with Treg cells were non-responsive. They were unable to proliferate when challenged with alloantigen (Fig. 2A). CD8+ cells were unable to produce IFN-γ (Fig.2B and 2C), and were unable to kill H-2^{b} target cells even after further stimulation *in vitro* (Fig.2D). This T cell non-responsiveness was antigen-specific. D2 T cells proliferated strongly in response to third party C3H H-2^{k} stimulator cells (Fig.2A).

In addition to documenting T cell non-responsiveness *in vitro* effects, we observed similar effects *in vivo.* Following transfer of Treg cells previously primed with H-2^{b} alloantigen, and then boosted with donor cells, mice were injected with CFSE-labeled donor and third party target cells and examined for the presence of these cells in the spleen. Pilot studies revealed that following immunization, there was a marked reduction of donor, but not third party target cells within 2 hours of injection (Fig.3A). However, in mice that had received Treg, similar numbers of CFSE-labeled donor target cells were observed in control and immunized mice. By contrast, in mice that had received Tcon, numbers of both donor and third party targets were markedly reduced. The reduction of third party target cells probably reflects the non-specific CTL activity associated with the vigorous CTL response to donor alloantigen. Table I indicates that the effects we observed were very similar in the 4 mice of each group. Since the *in vivo* CTL assay does not require the *in vitro* expansion, this approach is considered to be direct evidence of Treg function *in vivo* (Suvas, S., et al., J Exp Med 198:889 (2003)).

**TABLE I**

| Mice (n=4/group) Target Cells | | |
|---|---|---|
| | H-2^{b} (CFSE bright) | H-2^{k} (CFSE dim) |
| Immunized vs | | |
| Naive mice | | |
| No transfer | 74.4 ± 4.3% ** | 0.8 ± 0.02% |
| Tcon | 75.9 ± 4.1 %** | 61.2 ± 6.6%* |
| Treg | 0.2 ± 0.006% | 0.5 ± 0.01% |

| | | |
|---|---|---|
| The values shown indicate the killing of CFSE-labeled H-2^{b} or H-2^{k} target cells in the spleens of immunized mice determined by a formula indicated in materials and methods. The p values indicate significant differences between mice injected with Treg or T control, or immunized mice that did not receive cell transfer. * indicates p < 0.01), ** indicates p values < 0.001) | | |

### T cell non-responsiveness depends upon CD4+CD25+ cells that require continuous specific antigen- stimulation

The next series of experiments confirmed the requirement of CD4+CD25+ cells for the suppressive effects and revealed that continuous stimulation of specific antigen was needed to sustain T cell non-responsiveness. Groups of mice received a single injection of Treg or Tcon, or no cells. Some mice received booster injections of donor alloantigen every two weeks and others not injected served as controls. In animals that had received the booster injections, we observed a progressive increase in the splenic CD4+CD25+ cells during the next three months in animals that had received Tregs, but not in those that had received Tcon cells (Fig.4A). Since these mice were not lymphopenic, the increase could not be attributed to the homeostatic expansion of CD4+CD25+ cells described by others (Annacker, O., et al., J Immunol 166:3008 (2001)). This expansion was dependent upon continuous boosting with donor alloantigen. If at two months the mice received splenic cells from H-2^{k} C3H mice instead of H-2^{b} B6 cells, the numbers of CD4+CD25+ cells decreased to baseline values within one month (Fig.4B). Splenic CD8+CD25+ cells probably did not play a significant role since they comprised <1% of CD8+ cells in mice that had received Treg.

Continuous stimulation with specific antigen was required for Treg to sustain blockade of CTL activity. Figure 4C shows that the animals that had received Tregs and 3 to 5 subsequent booster immunizations of donor alloantigen for 2 to 3 months were unable to develop anti-H-2^{b} CTL activity. However, if injections of third party H-2^{k} cells instead of donor cells were given, the mice demonstrated strong anti-H-2^{b} CTL activity within one month (Figure 4D).

We next obtained evidence that the increased numbers of CD4+CD25+ cells in mice given Treg followed by booster immunizations of donor alloantigen expressed FoxP3 and were required for T cell non-responsiveness. Mice that received Treg, Tcon or no cells followed by booster immunizations every two weeks were sacrificed at 2 months. Although the total numbers of splenic CD4+ cells were similar in each of the groups, the CD4+CD25+ subset was significantly increased in mice that had received Treg (Table II). Examination of CD4+CD25+ and CD4+CD25- cells revealed that the CD25+ subset expressed significantly higher levels of FoxP3mRNA by real time PCR (Fig. 5B). Moreover, the number of CD4+CD25+ FoxP3+ cells quantified by flow cytometry was significantly increased in mice that had received Treg compared to those that received Tcon (Figs. 5C and 5D).

CD4+CD25+ cells were probably responsible for antigen-specific non-responsiveness to B6 alloantigens. As shown in Fig. 6A, depletion of CD25+ cells abolished the tolerogenic effect and adding back this subset restored the suppression. As with CTL activity, depletion of this CD25+ cells increased allo-CTL activity to levels similar to animals that had received Tcon. Again, adding back CD25+ Tregs in a 1:10 ratio restored suppressive activity (Fig. 6B). Since CD8+CD25+ cells comprised only 1% of total CD25+ cells, this suppressive effect was presumably to CD4+CD25+ cells. These experiments, however, do not exclude an effect of CD8+ suppressor cells.

**TABLE II**

| CD4+CD25+ cells at two months following cell transfer | | | | |
|---|---|---|---|---|
| T cell transfer | Spleen cells x 10⁶ (Mean ± SEM) | % CD4/spleen (Mean ± SEM) | % CD25/CD4+ (Mean ± SEM) | Total numbers of CD4+CD25+x10⁶ |
| Nil (n=2) | 89.5 ± 3.5 | 25 ± 2.0 | 7.5 ± 0.5 | 1.8 ± 0.2 |
| Tcon cells (n=4) | 94 ± 3.2 | 28.5 ± 2.3 | 7.4 ± 0.7 | 1.9 ± 0.2 |
| Treg cells (n=4) | 89.8 ± 3.1 | 27.7 ± 1.9 | 12.5 ± 1.1 | 3.3 ± 0.5 (*p<0.01) |

### Donor regulatory T cells educate recipient T cells to become tolerogenic CD4+CD25+ cells in vivo

To learn whether the increased CD4+CD25+ suppressor cells were the progeny of donor Tregs or derived from the recipient, the experiment was repeated using the protocol described above with Thy1.1 B6 mice serving as the source of the Tregs and congenic Thy1.2 mice as recipients. Here we again noted a progressive increase in CD4+CD25+ cells in B6 mice during the three months following a single injection of 8 million anti-H-2^{d} Treg and almost all cells were recipient Thy 1.2 origin (Fig. 7A). At one month only 1% of splenic T cells were stained by anti-Thy1.1 (results not shown). Thy 1.2 negative T cells were < 2% and these cells did not express CD25 (Fig. 7B). In comparison with Tcon, Tregs were enriched in cells expressing CD25, CD122 (IL-2R chain), CD103 (alpha E integrin) and GITR (Fig 7C and 7D.), and most of the CD122 and CD103 cells also expressed CD25 (Fig. 7C). See also Table III. Others have shown that TGF-β up-regulates CD103 expression (Cerwenka, A., et al., J Immunol 153:4367 (1994)).

**TABLE III**

| Phenotypic characterization of CD4+ cells at three months following T cell transfer | | | | |
|---|---|---|---|---|
| T cell transfer (n=3) | % CD25/CD4 | %CD122/CD4 | % CD103/CD4 | % GITR/CD4 |
| Nil | 12.3 ± 0.9 | 8 ± 0.5 | 7.9 ± 0.9 | 12.5 ± 0.8 |
| Tcon cells | 12 ± 1.2 | 10.2 ± 0.4 | 7.2 ± 0.5 | 15.6 ± 1.2 |
| Treg cells | 25.7 ± 1.0 (***) | 25.3 ± 0.6 (***) | 19.6 ± 1.3 (**) | 29.5 ± 5.3 (*) |

| | | | | |
|---|---|---|---|---|
| * indicates p<0.05; ** p<0.01; *** p<0.001 Mean ± SEM % CD4+ cells expressing CD25, CD122, CD103 and GITR in the Thy 1.2 gate (6 mice per group). P values indicate significant differences between Treg and Nil or Tcon. | | | | |

The functional properties of the educated mouse splenic CD4+CD25+ cells were similar to educated human peripheral blood CD4+ cells reported previously (Zheng, S. G., et al., J Immunol 172:5213 (2004)). While natural CD4+CD25+ cells and human natural-like CD4+CD25+ cells induced with TGF-β have cytokine-independent suppressive activity (Sakaguchi, S., et al., Immunol Rev 182:18 (2001); Piccirillo, C. A. and Shevach, E. M., Semin Immunol 16:81 (2004); Yamagiwa, S., et al., J Immunol 166:7282 (2001)), the suppressive activity of our educated CD4+CD25+ cells was abolished by either anti-TGF-β or anti-IL-10 (Fig. 8A). Of great interest, the anti-H-2^{d} suppressive activity of CD4+CD25+ cells from mice that had received Tregs was significantly greater than CD4+CD25+ cells from mice that had received Tcon. This effect was antigen-specific since anti-H-2^{d} CD4+CD25+ cells had minimal suppressive activity against H-2^{k} stimulator cells (Fig. 8B). These experiments were performed with a single source of CD25- responder cells and a ratio of CD4regs to CD4 responders of 1:6. At this ratio, the antigen non-specific suppressive activity of endogenous CD4+CD25+ regulatory cells on the response of CD4+ cells to allogeneic stimulator cells is diluted out (Fig. 8C).

### Discussion

In this study we observed that Tregs induced with IL-2 and TGF-β *ex vivo* can prolong the survival of heart allografts in completely MHC-mismatched mice without any additional immunosuppression. We also administered repeat allogeneic cell infusions in non-transplanted mice to investigate the mechanism of action. We observed that a single injection of T cells primed with allogeneic cells and TGF-β (Treg) followed by continuous boosts of alloantigen could induce long term antigen-specific non-responsiveness in the recipients. This tolerogenic effect appeared to be secondary to the ability of the transferred Tregs to educate donor CD4+ cells to become CD25+ cells.

It is recognized that TGF-β can induce both CD4+ and CD8+ cells to become suppressor cells. In 1994 we reported that human CD8+ cells activated with IL-2 and TGF-β became cytokine-dependent suppressors of T cell-dependent antibody production (Gray, J. D., et al., J Exp Med 180:1937 (1994)). We subsequently observed that TGF-β induced naïve CD4+ cells to become CD4+CD25+ cells with a phenotype and suppressive activities indistinguishable from the natural CD4+CD25+ cells described by others (Sakaguchi, S., et al., Immunol Rev 182:18 (2001); Piccirillo, C. A. and Shevach, E. M., Semin Immunol 16:81 (2004)). These cells had a contact-dependent, cytokine-independent mechanism of action and were potent inhibitors of CD8+ T cell activation (Yamagiwa, S., et al., J Immunol 166:7282 (2001); Piccirillo, C. A. and Shevach, E. M., J Immunol 167:1137 (2001)). We observed that TGF-β did not expand endogenous CD4+CD25+ cells, but induced CD4+CD25- cells to develop this function (Zheng, S. G., et al., J Immunol 172:5213 (2004)). Subsequently, Chen and co-workers reported that TGF-β induced mouse CD4+CD25- cells to become CD25+ suppressor cells that express FoxP3 (Chen, W., et al., J Exp Med 198:1875 (2003)), and our lab and others have confirmed this finding (Zheng, S. G., et al., J Immunol 172:5213 (2004); Fu, S., et al. Am J Transplant 4:1614 (2004); Schramm, C., et al., Int Immunol 16:1241 (2004); Park, H. B., et al., Int Immunol 16:1203 (2004); Fantini, M. C., et al., J Immunol 172:5149 (2004)). Prior work by Blazar and co-workers found that CD4+CD25- cells tolerized with IL-2 and TGF-β could increase survival in a model of alloantigen-induced graft versus host disease (Chen, Z. M., et al., Blood 101:5076 (2003)).

Since we had been able to induce CD8+ cells to become suppressor cells with TGF-β, and others had shown that CD8+ regulatory cells could suppress the rejection of heart allografts (Liu, J., et al., Transpl Immunol 13:239 (2004)), we utilized total T cell preparations containing both CD4+ and CD8+ cells in our initial study. In preliminary work using the mouse graft-versus-host disease model that we had used previously (Zheng, S. G., et al., J Immunol 172:1531 (2004), we found that the combination of CD4+ and CD8+ Tregs have more potent therapeutic effects than purified CD4+ Tregs (unpublished observations). In the present experiments, we observed only small numbers of CD8+CD25+ cells in the recipients, and less than 1% Thy1.1 congenic T cells remained in the recipients one month after transfer. Nonetheless, we cannot exclude the possibility that CD8+ Tregs induced with TGF-β contributed to the observed therapeutic effects.

In this study the number of CD4+CD25+ cells of recipient origin progressively increased in response to bi-weekly booster immunizations with allogeneic cells. Although CD25 is a marker of activated T cells, it is unlikely that the cells we observed are allogeneic effector cells. These cells were non-responsive to donor alloantigen. They blocked the ability of recipient T cells to proliferate and produce cytokines in response to donor alloantigens and they prevented CD8+ cells from developing CTL activity. Furthermore, the persistence of donor target cells in the *in vivo* CTL assay provides additional evidence of Treg activity *in vivo* as stated above. Finally, the evidence that these CD4+CD25+ cells express both FoxP3 mRNA and protein strongly suggests that the booster immunizations were expanding CD4+CD25+ regulatory cells *in vivo.*

Our results are consistent with other reports that CD4+CD25+ regulatory cells have a protective effect in transplant rejection. Van Maurik and colleagues have documented that CD4+CD25+ can markedly prolong survival of cardiac allografts, although these cells were induced by indirect methods (van Maurik, A., et al., J Immunol 169:5401 (2002)). Benghiat and co-workers have recently reported that natural CD25+ Treg cells control Th1- and Th2-type allo-helper T-cell responses (Benghiat, F.S., et al., Trnasplantation 79:648 (2005)). Both of these groups have reported that the protective CD4+CD25+ cells require continuous antigen stimulation (Cobbold, S. P., et al., Transpl Int 16:66 (2003); Thorstenson, K. M. and Khoruts, A., J Immunol 167:188 (2001)). Schenk and co-workers have reported that depletion of CD4+CD25+ cells markedly accelerates acute rejection of heart allografts rejection (Schenk, S., et al., J Immunol 174:3741 (2005)). Because epitope spreading in alloreactive cells may contribute to chronic rejection (Ciubotariu, R., et al., J Clin Invest 101:398 (1998)), Salama and co-workers have suggested that CD4+CD25+ cells may limit this effect and thus have a protective role (Salama, A. D., et al., J Am Soc Nephrol 14:1643 (2003)).

While some research has shown that polyclonal CD4+CD25+ cells can educate other CD4+ cells to become suppressor cells *in vitro* (Jonuleit, H., et al., J Exp Med 196:255 (2002); Dieckmann, D., et al., J Exp Med 196:247-53 (2002)), others have used indirect methods to achieve infectious tolerance *in vivo* (Qin, S., et al., Science 259:974 (1993)). This is the first demonstration that Tregs induced ex-*vivo* can educate recipient CD4+ cells to become CD25+ cells that have similar suppressive activity. Thus, this TGF-β induced regulatory T cells act more like a vaccine than a conventional adoptive therapy. They appear to prevent organ graft rejection by eliciting an active, protective immune response in the recipient.

Examination of the functional properties of CD4+CD25+ cells harvested from the tolerized recipients revealed that their mechanism of action could be blocked by either anti-TGF-β or anti-IL-10. This result is consistent to a study of human CD4+CD25+ regulatory cells induced with TGF-β. We reported that anti-TGF-β was unable to block the suppressive effects of naive CD4+ cells induced *ex-vivo* to become alloantigen-specific suppressor cells. Nonetheless, these cells produced both TGF-β and IL-10 following restimulation, and both of these cytokines were necessary for these CD4+CD25+ Tregs to induce other CD4+CD25- to become suppressor cells. Moreover, the suppressive effects of the secondary CD4+CD25+ Tregs were blocked by either anti-TGF-β or anti-IL-10. Thus, the transfer of CD4+CD25+ Tregs with cytokine-independent suppressive effects *in vitro* may result in cytokine-dependent suppressive effects *in vivo.* In experimental models of immune-mediated disease in mice, the role of TGF-β and IL-10 in supporting the suppressive effects of CD4+CD25+ cells has been established (Coombes, J. L., et al., Immunol Rev 204:184 (2005); Peng, Y., et al., Proc Natl Acad Sci U S A 101:4572 (2004)).

In this study we observed long term survival in some, but not all, of the allogeneic heterotopic heart transplants. Although we observed prolonged graft survival, only two of these six grafts survived to 100 days, making it highly unlikely that *in vivo* tolerance was achieved. By contrast, animals that received booster immunizations of donor alloantigen could not mount a response to donor cells. The results of these experiments infer that in the attempt to establish a tolerant state, there is a probable requirement for persistent antigenic stimulation to sustain the activity of the Tregs which may not sufficiently exist after heart transplantation alone with a presumed paucity of donor antigens being shed. Histology of the hearts from animals sacrificed 100 days post-transplantation did not show classic pathologic evidence of acute or chronic rejection. However, despite intact function, the myocardium did have a moderate monocytic infiltrate. Unfortunately, we did not save frozen tissue at the time of the original experiments, so that we cannot evaluate if there was FoxP3 mRNA displayed by these mononuclear cells, and therefore cannot exclude the possibility that this mononuclear infiltrate may be an atypical manifestation of chronic rejection. Further experiments in which animals receive additional injections of relevant donor MHC alloantigens may improve the graft survival results observed, as well as decrease the observed mononuclear infiltrates. The use of TGF-β treated T cells generated *ex-vivo* to alter the recipient's immune system to develop a dominant regulatory response rather than an alloreactive one offers a novel therapeutic strategy for clinical organ transplantation.

## Claims

1. An *ex vivo* population of donor alloactivated regulatory recipient T cells (Tregs) for use in a method for preventing rejection of donor tissue in a recipient, wherein the method comprises:
(a) producing, *ex vivo,* a population of donor alloactivated regulatory recipient T cells (Tregs);
(b) introducing a first dose of said recipient Tregs into said recipient;
(c) transplanting donor tissue into said recipient; and
(d) introducing donor antigen into said recipient after transplantation of the donor tissue.

2. Use of an *ex vivo* population of donor alloactivated regulatory recipient T cells (Tregs) for the preparation of a medicament for preventing rejection of donor tissue in a recipient, wherein the administration pattern of the medicament comprises (a) introducing a first dose of said recipient Tregs into said recipient and (b) introducing donor antigen into said recipient after transplantation of the donor tissue.

3. An *ex vivo* population of donor alloactivated regulatory recipient T cells (Tregs) for preventing rejection of donor tissue in a recipient, wherein the administration pattern of the medicament comprises (a) introducing a first dose of said recipient Tregs into said recipient and (b) introducing donor antigen into said recipient after transplantation of the donor tissue.

4. The use or composition according to any one of claims 1 to 3 wherein said first dose of recipient Tregs is introduced at least one day before transplantation.

5. The use or composition according to any one of claims 1 to 3 wherein said donor antigen is introduced to said recipient on a periodic basis.

6. The use or composition according any one of claims 1 to 3 wherein said donor antigen is introduced on a periodic basis.

7. The use or composition according to any one of claims 1 to 3 wherein said donor antigen is a histocompatability antigen.

8. The use or composition according to any one of claims 1 to 3 wherein a second dose of recipient Tregs is introduced after transplantation.

9. The use or composition according to any one of claims 1 to 3 wherein a second dose of recipient Tregs is introduced from one to five days after transplantation.

10. The use or composition according to any one of claims 1 to 3 wherein the producing of said Tregs comprises: isolating recipient peripheral blood mononuclear cells (PBMC); and contacting *ex vivo* said recipient PBMCs with donor antigen and a regulatory composition comprising TGFβ to form said recipient Tregs.

11. The use or composition according to claim 10 wherein said regulatory composition further comprises IL-2, IL-4, IL-10 and/or IL-15.

12. The use or composition according to claim 10 wherein donor cells are used as said donor antigen.

13. The use or composition according to claim 12 wherein said donor cells are not T cells.

14. The use or composition according to claim 10 wherein donor PBMCs are used as said donor antigen.

15. The use or composition according to claim 12 wherein said donor cells comprise spleen cells or irradiated PBMCs.

16. The use or composition according to any one of claims 1 to 3 wherein said donor tissue is selected from the group consisting of heart, lung, liver, kidney, intestine pancreas and pancreatic islet cells.

17. The use or composition according to claim 16 wherein said donor tissue is a heart.

18. The use or composition according to any one of claims 1 to 3 wherein said introducing of step (d) further comprises introducing a second dose of said recipient Tregs into said recipient.

19. An *ex vivo* population of donor alloactivated regulatory recipient T cells (Tregs) for use in a method for preventing rejection of a heart transplant in a recipient, wherein the method comprises:
(a) producing, *ex vivo*, a population of donor alloactivated regulatory recipient T cells (Tregs);
(b) introducing a first dose of said recipient Tregs into said recipient;
(c) transplanting donor tissue into said recipient; and
(d) introducing donor antigen into said recipient after transplantation of the heart transplant.

20. Use of an *ex vivo* population of donor alloactivated regulatory recipient T cells (Tregs) for the preparation of a medicament for preventing rejection of a heart transplant in a recipient, wherein the administration pattern of the medicament comprises (a) introducing a first dose of said recipient Tregs into said recipient and (b) introducing donor antigen into said recipient after transplantation of the heart.

21. An *ex vivo* population of donor alloactivated regulatory recipient T cells (Tregs) for preventing rejection of a heart transplant in a recipient, wherein the administration pattern of the medicament comprises (a) introducing a first dose of said recipient Tregs into said recipient and (b) introducing donor antigen into said recipient after transplantation of the heart.

22. The use or composition according to claim 19 or claim 20 wherein said producing comprises:
isolating recipient peripheral blood mononuclear cells (PBMC); and contacting *ex vivo* said recipient PBMCs with donor antigen and a regulatory composition comprising TGFβ to form said recipient Tregs.

23. The use or composition according to any one of claims 19 to 21 wherein said regulatory composition further comprises IL-2.

24. The use or composition of any one of the preceding claims wherein said recipient is a human.

## Patentansprüche

1. Ex-vivo-Population von spenderalloaktivierten regulatorischen Empfänger-T-Zellen (Tregs) zur Verwendung in einem Verfahren zur Unterbindung der Abstoßung von Spendergewebe bei einem Empfänger, worin das Verfahren Folgendes umfasst:
(a) das Herstellen einer Population von spenderalloaktivierten regulatorischen Empfänger-T-Zellen (Tregs) *ex vivo,*
(b) das Zuführen einer ersten Dosis der Empfänger-Tregs in den Empfänger,
(c) das Transplantieren von Spendergewebe in den Empfänger und
(d) das Zuführen von Spenderantigen an den Empfänger nach der Transplantation des Spendergewebes.

2. Verwendung einer Ex-vivo-Population von spenderalloaktivierten regulatorischen Empfänger-T-Zellen (Tregs) zur Herstellung eines Medikaments zur Unterbindung der Abstoßung von Spendergewebe bei einem Empfänger, worin das Verabreichungsmuster des Medikaments (a) das Zuführen einer ersten Dosis der Empfänger-Tregs an den Empfänger und (b) das Zuführen von Spenderantigen an den Empfänger nach der Transplantation des Spendergewebes umfasst.

3. Ex-vivo-Population von spenderalloaktivierten regulatorischen Empfänger-T-Zellen (Tregs) zur Unterbindung der Abstoßung von Spendergewebe bei einem Empfänger, worin das Verabreichungsmuster des Medikaments (a) das Zuführen einer ersten Dosis der Empfänger-Tregs an den Empfänger und (b) das Zuführen von Spenderantigen an den Empfänger nach der Transplantation des Spendergewebes umfasst.

4. Verwendung oder Zusammensetzung nach einem der Ansprüche 1 bis 3, worin die erste Dosis der Empfänger-Tregs zumindest einen Tag vor der Transplantation zugeführt wird.

5. Verwendung oder Zusammensetzung nach einem der Ansprüche 1 bis 3, worin das Spenderantigen dem Empfänger periodisch zugeführt wird.

6. Verwendung oder Zusammensetzung nach einem der Ansprüche 1 bis 3, worin das Spenderantigen periodisch zugeführt wird.

7. Verwendung oder Zusammensetzung nach einem der Ansprüche 1 bis 3, worin das Spenderantigen ein Histokompatibilitätsantigen ist.

8. Verwendung oder Zusammensetzung nach einem der Ansprüche 1 bis 3, worin eine zweite Dosis der Empfänger-Tregs nach der Transplantation zugeführt wird.

9. Verwendung oder Zusammensetzung nach einem der Ansprüche 1 bis 3, worin eine zweite Dosis der Empfänger-Tregs einen bis fünf Tage nach der Transplantation zugeführt wird.

10. Verwendung oder Zusammensetzung nach einem der Ansprüche 1 bis 3, worin das Herstellen der Tregs Folgendes umfasst: das Isolieren von mononukleären Zellen aus dem peripheren Blut (PBMC) des Empfängers und das Kontaktieren der Empfänger-PBMC *ex vivo* mit Spenderantigen und einer regulatorischen Zusammensetzung, die TGFβ umfasst, um so die Empfänger-Tregs zu bilden.

11. Verwendung oder Zusammensetzung nach Anspruch 10, worin die regulatorische Zusammensetzung weiter IL-2, IL-4, IL-10 und/oder IL-15 umfasst.

12. Verwendung oder Zusammensetzung nach Anspruch 10, worin Spenderzellen als das Spenderantigen verwendet werden.

13. Verwendung oder Zusammensetzung nach Anspruch 12, worin die Spenderzellen nicht T-Zellen sind.

14. Verwendung oder Zusammensetzung nach Anspruch 10, worin Spender-PBMC als das Spenderantigen verwendet werden.

15. Verwendung oder Zusammensetzung nach Anspruch 12, worin die Spenderzellen Milzzellen oder bestrahlte PBMC umfassen.

16. Verwendung oder Zusammensetzung nach einem der Ansprüche 1 bis 3, worin das Spendergewebe aus der aus Herz, Lunge, Leber, Niere, Darm, Bauchspeicheldrüse und Langerhans-Inselzellen bestehenden Gruppe ausgewählt ist.

17. Verwendung oder Zusammensetzung nach Anspruch 16, worin das Spendergewebe ein Herz ist.

18. Verwendung oder Zusammensetzung nach einem der Ansprüche 1 bis 3, worin das Zuführen nach Schritt (d) weiters das Zuführen einer zweiten Dosis der Empfänger-Tregs an den Empfänger umfasst.

19. Ex-vivo-Population von spenderalloaktivierten regulatorischen Empfänger-T-Zellen (Tregs) zur Verwendung in einem Verfahren zur Unterbindung der Abstoßung eines Herztransplantats bei einem Empfänger, worin das Verfahren Folgendes umfasst:
(a) das Herstellen einer Population von spenderalloaktivierten regulatorischen Empfänger-T-Zellen (Tregs) *ex vivo,*
(b) das Zuführen einer ersten Dosis der Empfänger-Tregs in den Empfänger,
(c) das Transplantieren von Spendergewebe in den Empfänger und
(d) das Zuführen von Spenderantigen an den Empfänger nach der Transplantation des Herztransplantats.

20. Verwendung einer Ex-vivo-Population von spenderalloaktivierten regulatorischen Empfänger-T-Zellen (Tregs) zur Herstellung eines Medikaments zur Unterbindung der Abstoßung eines Herztransplantats bei einem Empfänger, worin das Verabreichungsmuster des Medikaments (a) das Zuführen einer ersten Dosis der Empfänger-Tregs an den Empfänger und (b) das Zuführen von Spenderantigen an den Empfänger nach der Transplantation des Herzens umfasst.

21. Ex-vivo-Population von spenderalloaktivierten regulatorischen Empfänger-T-Zellen (Tregs) zur Unterbindung der Abstoßung eines Herztransplantats bei einem Empfänger, worin das Verabreichungsmuster des Medikaments (a) das Zuführen einer ersten Dosis der Empfänger-Tregs an den Empfänger und (b) das Zuführen von Spenderantigen an den Empfänger nach der Transplantation des Herzens umfasst.

22. Verwendung oder Zusammensetzung nach Anspruch 19 oder Anspruch 20, worin das Herstellen Folgendes umfasst: das Isolieren von mononukleären Zellen aus dem peripheren Blut (PBMC) des Empfängers und das Kontaktieren der Empfänger-PBMC *ex vivo* mit Spenderantigen und einer regulatorischen Zusammensetzung, die TGFβ umfasst, um so die Empfänger-Tregs zu bilden.

23. Verwendung oder Zusammensetzung nach einem der Ansprüche 19 bis 21, worin die regulatorische Zusammensetzung weiters IL-2 umfasst.

24. Verwendung oder Zusammensetzung nach einem der vorangegangenen Ansprüche, worin der Empfänger ein Mensch ist.

## Revendications

1. Population ex *vivo* de cellules T de receveur régulatoires de donneur alloactivées (Tregs) pour utilisation dans une méthode pour prévenir le rejet d'un tissu de donneur chez un receveur, où la méthode comprend:
(a) produire, ex *vivo,* une population de cellules T de receveur régulatoires de donneur alloactivées (Tregs);
(b) introduire une première dose desdites Tregs de receveur dans ledit receveur;
(c) transplanter le tissu du donneur dans ledit receveur; et
(d) introduire l'antigène du donneur dans ledit receveur après la transplantation du tissu du donneur.

2. Utilisation d'une population ex *vivo* de cellules T de receveur régulatoires de donneur alloactivées (Tregs) pour la préparation d'un médicament pour prévenir le rejet du tissu du donneur dans un receveur, où le schéma d'administration du médicament comprend (a) introduire une première dose desdites Tregs de receveur dans ledit receveur et (b) introduire l'antigène du donneur dans ledit receveur après la transplantation du tissu du donneur.

3. Population ex *vivo* de cellules T de receveur régulatoires de donneur alloactivées (Tregs) pour la prévention du rejet du tissu du donneur dans un receveur, où le schéma d'administration du médicament comprend (a) introduire une première dose desdites Tregs de receveur dans ledit receveur et (b) introduire l'antigène du donneur dans ledit receveur après la transplatation du tissu du donneur.

4. Utilisation ou composition selon l'une quelconque des revendications 1 à 3, où ladite première dose de Tregs de receveur est introduite au moins un jour avant la transplantation.

5. Utilisation ou composition selon l'une quelconque des revendications 1 à 3, où ledit antigène du donneur est introduit dans ledit receveur sur une base périodique.

6. Utilisation ou composition selon l'une quelconque des revendications 1 à 3, où ledit antigène du donneur est introduit sur une base périodique.

7. Utilisation ou composition selon l'une quelconque des revendications 1 à 3, où ledit antigène de donneur est un antigène d'histocompatabilité.

8. Utilisation ou composition selon l'une quelconque des revendications 1 à 3, où une deuxième dose des Tregs de receveur est introduite après la transplantation.

9. Utilisation ou composition selon l'une quelconque des revendications 1 à 3, où une deuxième dose de Tregs de receveur est introduite de un à cinq jours après la transplantation.

10. Utilisation ou composition selon l'une quelconque des revendications 1 à 3, où la production desdites Tregs comprend: isoler des cellules mononucléaires de sang périphérique de receveur (PBMC); et mettre en contact ex vivo lesdites PBMCs de receveur avec l'antigène du donneur et une composition régulatoire comprenant TGFβ pour former lesdites Tregs de receveur.

11. Utilisation ou composition selon la revendication 10, où ladite composition régulatoire comprend en outre IL-2, IL-4, IL-10 et/ou IL-15.

12. Utilisation ou composition selon la revendication 10, où les cellules du donneur sont utilisées comme ledit antigène du donneur.

13. Utilisation ou composition selon la revendication 12, où lesdites cellules du donneur ne sont pas des cellules T.

14. Utilisation ou composition selon la revendication 10, où des PBMCs de donneur sont utilisées comme antigène de donneur précité.

15. Utilisation ou composition selon la revendication 12, où lesdites cellules du donneur comprennent des cellules spléniques ou des PBMCs irradiées.

16. Utilisation ou composition selon l'une quelconque des revendications 1 à 3, où ledit tissu de donneur est sélectionné dans le groupe consistant en coeur, poumon, foie, rein, intestin, pancréas et cellules d'îlots pancréatiques.

17. Utilisation ou composition selon la revendication 16, où ledit tissu du donneur est un coeur.

18. Utilisation ou composition selon l'une quelconque des revendications 1 à 3, où ladite introduction de l'étape (d) comprend en outre l'introduction d'une deuxième dose desdites Tregs de receveur dans ledit receveur.

19. Population ex *vivo* de cellules T de receveur régulatoires de donneur alloactivées (Tregs) pour utilisation dans une méthode pour prévenir le rejet d'une transplantation du coeur chez un receveur, où la méthode comprend:
(a) produire, *ex vivo,* une population de cellules T de receveur régulatoires de donneur alloactivées (Tregs);
(b) introduire une première dose desdites Tregs de receveur dans ledit receveur;
(c) transplanter le tissu du donneur dans ledit receveur; et
(d) introduire l'antigène du donneur dans ledit receveur après la transplantation de la greffe cardiaque.

20. Utilisation d'une population *ex vivo* de cellules T de receveur régulatoires de donneur alloactivées (Tregs) pour la préparation d'un médicament pour prévenir le rejet d'une greffe cardiaque chez un receveur, où le schéma d'administration du médicament comprend (a) l'introduction d'une première dose desdites Tregs de receveur dans ledit receveur et (b) l'introduction de l'antigène du donneur dans ledit receveur après la transplantation du coeur.

21. Population *ex vivo* de cellules T de receveur régulatoires de donneur alloactivées (Tregs) pour la prévention du rejet d'une greffe cardiaque chez un receveur, où le schéma d'administration du médicament comprend (a) introduire une première dose desdites Tregs de receveur dans ledit receveur et (b) introduire l'antigène du donneur dans ledit receveur après la transplantation du coeur.

22. Utilisation ou composition selon la revendication 19 ou la revendication 20, où ladite production comprend:
isoler des cellules mononucléaires de sang périphérique de receveur (PBMC); et mettre en contact *ex vivo* lesdites PBMCs de receveur avec l'antigène du donneur et une composition régulatoire comprenant TGFβ pour former lesdites Tregs de receveur.

23. Utilisation ou composition selon l'une quelconque des revendications 19 à 21, où ladite composition régulatoire comprend en outre IL-2.

24. Utilisation ou composition selon l'une quelconque des revendications précédentes, où ledit receveur est un humain.
